Europäisches Patentamt

European Patent Office

Office européen des brevets

(11) Publication number: **0 170 247**
**B1**

(12) **EUROPEAN PATENT SPECIFICATION**

(45) Date of publication of patent specification: 26.09.90

(21) Application number: 85109515.8

(22) Date of filing: 29.07.85

(51) Int. Cl.⁵: **A 61 K 9/50**, A 61 K 37/14,
A 61 K 31/375, A 61 K 31/195

(54) Hemoglobin-containing liposomes and process for preparing the same.

(30) Priority: 31.07.84 JP 159281/84

(43) Date of publication of application:
05.02.86 Bulletin 86/06

(45) Publication of the grant of the patent:
26.09.90 Bulletin 90/39

(84) Designated Contracting States:
BE DE FR GB NL SE

(56) References cited:
GB-A- 658 060

CHEMICAL ABSTRACTS, vol. 101, 1984, pages
356-357, no. 36646h, Columbus, Ohio, US; J.
SZEBENI et al.: "Oxidative interactions between
hemoglobin and membrane lipid. A lipsome
model", & BIOCHEM. J. 1984, 220 (3), 685-92

The file contains technical information
submitted after the application was filed and
not included in this specification

(73) Proprietor: TERUMO KABUSHIKI KAISHA
trading as TERUMO CORPORATION
44-1, 2-chome, Hatagaya Shibuya-Ku
Tokyo 151 (JP)

(72) Inventor: Suzuki, Kazuhiko
421-3, Kuroda
Fujinomiya-shi Shizuoka-ken (JP)
Inventor: Miyauchi, Yuji
1623-2, Aza-Shichogawara Matsuoka
Fuji-shi Shizuoka-ken (JP)

(74) Representative: Henkel, Feiler, Hänzel und
Partner
Möhlstrasse 37
D-8000 München 80 (DE)

(56) References cited:

BIOCHEMISTRY, vol. 24, no. 12, 4th June 1985,
pages 2827-2832, American Chemical Society,
US; J. SZEBENI et al.: "Encapsulation of
hemoglobin in phospholipid liposomes:
characterization and stability"
Biochem J (1984) 220(3), 685-692

Courier Press, Leamington Spa, England.

**Description**

Background of the invention
Field of the invention

The present invention relates to liposomes which contain hemoglobin and a process for preparing the same.

The liposomes of the invention are useful as artificial blood of a high oxygen-carrying capacity.

Description of the prior art

It is known that hemoglobin-containing liposomes are prepared by a so-called thin-film process. The hemoglobin-containing liposomes have been prepared by dissolving a liposome-forming lipid material in an appropriate organic solvent such as chloroform, removing the solvent from the resulting solution by distillation to form thin film of the lipid material, adding a hemoglobin solution to the thin film, vigorously stirring the mixture to form multilayer liposomes and subjecting the same to ultrasonic treatment (U.S. Patent No. 4,133,874). Such preparative method has an advantage in that there is associated with relatively little degeneration of the hemoglobin because of a shorter period of contact time of the hemoglobin with oxygen. On the other hand, however, it is not suitable for the production of a large amount of the hemoglobin due to low yield and difficulty in constructing equipment for a large-scale production.

We have investigated on preparing hemoglobin-containing liposomes using the dialysis process which is a process suitable for production on a large scale. As the dialysis process needs only simple manufacturing equipment and can be operated by a continuous method, it is suitable for the manufacturing on a large scale. On the other hand, however, it requires a longer period of operation time thereby increasing contact of the hemoglobin with oxygen. Accordingly, there is higher possibility for the heme iron in hemoglobin to be oxidized to produce methemoglobin thereby losing the oxygen-carrying capacity.

In the blood cell, even if hemoglobin is converted to the met form by an oxidation, it is reducible to the original form by the action of enzyme. However, conversion of hemoglobin to the met form outside the blood cell after hemolysis is necessarily associated with loss of the oxygen-carrying capacity.

Biochem. J. (1984), 220, 685—692 describes the oxidative interactions between the molecules of hemoglobin and the phospholipids of the membrane. According to said citation it was found that the oxidation of hemoglobin is parallel to the membrane lipid peroxidation and that these oxidations can largely be abolished by the use of a freshly prepared hemolysate instead of purified hemoglobin or by the use of saturated phosphatidylcholines by the preparation of the hemosomes. Furthermore it is described that in hemosomes prepared from purified hemoglobin and egg lecithin, alpha-tocopherol, catalase and ascorbate each protected against oxidation and peroxidation. However, this protecting effect is not sufficient. At page 688 the effects of additives on hemoglobin oxidation in hemosomes are described. Therein at line 4 to 7 it is stated that the addition of ascorbate is only initially inhibitory but after 6 hours this effect is lost. This clearly demonstrates that the hemosomes described in that document are not storable over longer periods and thus are not apt for clinical use.

Objects of the invention

It is an object of the invention to provide hemoglobin-containing liposomes in which oxidation of the hemoglobin is inhibited.

It is a further object of the invention to provide a process for preparing the above-mentioned hemoglobin-containing liposome which is suitable for the production on a large scale.

Detailed description of the invention

The present invention comprises liposomes which encapsulate hemoglobin and a methemoglobin formation-inhibiting agent and have a membrane composed of a lipid material. The liposome is defined as closed vehicles having a membrane principally composed of a lipid material.

The membrane has a bimolecular film structure and has an average thickness of 5 nm (50 Å).

Any lipid substances may be used in the invention for forming the membrane of liposomes so far as they can form liposomes. Both natural and synthetic lipid may be employed. Particularly preferable is phospholipid. As examples of the phospholipid are mentioned lecithin, phosphatidylethanolamine, phosphatidic acid, phosphatidylserin, phosphatidylinositol, phosphatidylglycerol, sphingomyelin, cardiolipin and products therefrom by hydrogenation according to a conventional method. Combinations of them may be used.

As the hemoglobin, there may be employed one obtained from red blood cells by hemolysis according to conventional methods.

Although there is no specific limitation in the methemoglobin formation-inhibiting agents, they must be non-toxic due to their use as artificial blood. Preferably employed are salts of ascorbic acid and glutathion. Suitable salts of ascorbic acid include the salts with an alkali metal such as lithium, potassium and sodium. Concentration of the hemoglobin contained in the liposomes is not critical, but is usually in the range from 5 to 45% by weight. The methemoglobin formation-inhibiting agent is used in an amount necessary for inhibiting hemoglobin oxidation which is generally in the range from 2 to 10 times the molar amount of hemoglobin charged, although it depends upon the nature of the agent. It is suitable that diameter of the liposomes is in the range approximately from 0.1 to 1.0 μm.

The liposomes of the invention are prepared by dissolving a liposome membrane-forming lipid material and a surface active agent in an organic solvent, removing the solvent from the resulting solution, adding to the residue a solution of hemoglobin to which a methemoglobin formation-inhibiting agent has been added and subjecting the resulting starting solution to dialysis with physiological saline solution containing the same methemoglobin formation-inhibiting agent to remove the surface active agent.

The process for preparing the liposomes according to the present invention is carried out by so-called dialysis method which is known per se. As the liposome membrane-forming lipid material are used those which are described above. The surface-active agent should carefully be selected so as not to degenerate the hemoglobin. As examples of preferred surface-active agents are mentioned salts of cholic acid, e.g., sodium cholate and sodium deoxycholate, block-copolymers of polyoxyethylene and polyoxyproylene, e.g., Pluronic F68 (product of Asahi Electro-Chemical Co., Ltd.) and polyethylene glycol p-octylphenyl ether, e.g., Triton X-100 (product of Eastman Kodak Co.).

In order to strengthen the membrane structure or to adjust the disintegration time, such substances as sterols, and electric charge donors may be employed, if desired, as constituents of the liposome membrane. As examples of the sterol are mentioned cholesterol, which is arranged so as to fill a gap of other constituents and has a function of stabilizing liposomes. As examples of the electric charge donor are mentioned electric charge substances of fatty acids having 14 or more carbon atoms, for example, stearic acid, oleic acid, linolic acid and linolenic acid.

The organic solvent for dissolving the constituents of the liposome membrane is usually chloroform or ethanol, but is not limited thereto. The organic solvent is removed from the solution of the liposome-membrane constituents by a conventional method as distillation, a physiological saline solution of hemoglobin to which a methemoglobin formation-inhibiting agent has been added is added to the residue, and the mixture is desirably subjected to such a treatment as ultrasonic treatment to form a homogeneous system. The starting solution thus obtained is subjected to dialysis according to a conventional method. As the dialysis solution is used a physiological saline solution to which the above-mentioned methemoglobin formation-inhibiting agent has been added. The surface-active agent is removed from the starting solution by the dialysis to produce liposomes in which the hemoglobin and the methemoglobin formation-inhibiting agent is incorporated. The liposomes thus obtained are of a uniform size adjusted to a diameter in the range from 0.1 to 1.0 μm depending upon the rate of dialysis. The dialytic process may be carried out either batchwise in a flat membrane cell with a dialytic membrane in which the starting solution is placed or continuously by means of external circulation using a hollow module.

The hemoglobin-containing liposomes of the invention are used as artificial blood in the same way as with the known ones; a suspension of the liposomes of the invention in physiological saline solution or substitute plasma is used as artificial blood. The liposome of the invention may be lyophilized for storage and dispersed before use in a physiological saline solution or substitute plasma.

The invention will be described in more details with reference to the following Examples and Test Examples.

Example 1

In a mixed solvent composed of chloroform and methyl alcohol are dissolved 1.211 g of eggyolk lecithin, 0.273 g of cholesterol and 2.156 g of sodium cholate (surface active agent). Then, the solvent is removed from said solution on an evaporator. To the residue is added 60 ml of hemolyzed hemoglobin (concentration 14.4 g/dl) to which 0.03 g of sodium ascorbate has been added, and the mixture is subjected to ultrasonic treatment (135 watts, 20 min) to give a uniform system. The resulting starting solution is placed in a flat membrane cell covered in the opening with dialytic membrane. The flat membrane cell is placed in a vessel equipped with stirrer blades, which is filled with a dialysis solution. A dialysis is conducted under stirring, while stirring the starting solution with a rotor and a stirrer. As the dialysis solution is used a physiological saline solution to which sodium ascorbate has been added (0.05% w/v). The dialysis is carried out at room temperature (25—28°C) for 22 hours to remove the sodium cholate thereby producing liposomes containing hemoglobin and sodium ascorbate.

Measurement was made of the hemoglobin-containing liposomes for concentration of methemoglobin therein (immediately after prepared) to find that it was 0.9%. On the contrary, concentration of methemoglobin in the hemoglobin-containing liposomes prepared in the same way as in Example 1 except that no methemoglobin formation-inhibiting agent was employed (control liposomes) was 40—50%. Furthermore, concentrations (%) of methemoglobin in the liposomes kept at 4°C for 1—4 weeks are shown in Table 1 both for the liposomes of Example 1 and the control liposomes. The methemoglobin concentration was measured by means of a CO oxymeter (manufactured by Instrumentation Laboratory).

TABLE 1

| Sample | Methemoglobin concentration (%) | | | |
|---|---|---|---|---|
| | After 1 wk | After 2 wks | After 3 wks | After 4 wks |
| Liposomes of Example 1 | 1.0 | 1.0 | 2.1 | 2.1 |
| Control liposomes | 60—70 | 70 | 70—80 | 80—90 |

Example 2

In a mixed solvent composed of chloroform and methyl alcohol are dissolved 3.0275 g of eggyolk lecithin, 0.6825 g of cholesterol and 5.389 g of sodium cholate. Then, the solvent is removed from said solution on an evaporator. To the residue is added 130 ml of hemolyzed hemoglobin (concentration 14.4 g/dl) to which 0.065 g of sodium ascorbate has been added. The mixture is subjected to ultrasonic treatment (135 watts, 20 min) to produce a uniform system. Dialysis is made of the starting solution thus obtained by an external circulation dialysis process by means of a hollow module, while stirring the starting solution by a rotor and a stirrer. As the dialysis solution is employed a physiological saline solution to which sodium ascorbate has been added (0.05% w/v). The dialysis is carried out at room temperature (25—28°C) for 8 hours to remove the sodium cholate. There are produced liposomes containing hemoglobin and sodium ascorbate.

The liposomes of the present invention comprise hemoglobin-containing liposomes which comprise liposomes with a membrane consisting of a lipid material in which hemoglobin and a methemoglobin formation-inhibiting agent are incorporated. They have excellent oxygen-carrying capacity because of protection of the hemoglobin from oxidation. In general, hemoglobin-containing liposomes are contacted with oxygen during preparation or storage of the product to result in the oxidation to methemoglobin, thereby reducing or losing the oxygen-carrying capacity. On the contrary, the oxidation of hemoglobin is inhibited in the liposomes of the invention in which a methemoglobin formation-inhibiting agent is coexisting so that they are of excellent properties as artificial blood.

In addition, the process for preparing hemoglobin-containing liposomes according to the invention, in which the liposomes are formed by dialysis, needs simple manufacturing equipment and is suitable for the production on a large scale.

Furthermore, there are obtained liposomes more uniform in size by the process according to the invention than by the thin film process. Variation in size of liposomes causes disadvantages when used as artificial red blood cell; the presence of liposomes with too large sizes will obstruct blood vessels to cause thrombosis, whereas liposomes of too small sizes will pass through the wall of blood vessels. Therefore, there are provided liposomes suitable for artificial red blood cell by the process of the invention.

Moreover, as the dialysis is made in the presence of a methemoglobin formation-inhibiting agent, the oxidation to the met form during the preparative process is also inhibited.

**Claims**

1. Hemoglobin-containing liposomes obtainable by dissolving a liposome membrane-forming lipid material and a surface-active agent in an organic solvent, removing the solvent from said solution, adding to the residue a physiological saline solution of hemoglobin to which a methemoglobin formation-inhibiting agent has been added characterized in that the starting solution thus obtained is subjected to dialysis with a physiological saline solution containing the same methemoglobin formation-inhibiting agent to remove the surface-active agent from the starting solution, thereby encapsulating the hemoglobin and the methemoglobin formation-inhibiting agent in the liposomes with membranes comprising the lipid material.

2. Liposomes according to Claim 1 wherein the lipid material is a phospholipid material selected from lecithin, phosphatidylethanolamine, phosphatidic acid, phosphatidylserin, phosphatidylinositol, phosphatidylglycerol, sphingomyelin, cardiolipin and hydrogenation products thereof.

3. Liposomes according to Claim 1 or 2 wherein the methemoglobin formation-inhibiting agent is a salt of ascorbic acid or glutathion.

4. Liposomes according to Claims 1—3 wherein concentration of the hemoglobin contained in the liposomes is in the range from 5 to 45% (w/w) and an amount of the methemoglobin formation-inhibiting agent used is in the range from 2 to 10 times the molar amount of the hemoglobin.

5. Process for preparing hemoglobin-containing liposomes which comprises dissolving a liposome membrane-forming lipid material and a surface-active agent in an organic solvent, removing the solvent from said solution, adding to the residue a physiological saline solution of hemoglobin to which a methemoglobin formation-inhibiting agent has been added and subjecting the starting solution thus obtained to dialysis with a physiological saline solution containing the same methemoglobin formation-inhibiting agent to remove the surface-active agent from the starting solution, thereby encapsulating the hemoglobin and the methemoglobin formation-inhibiting agent in the liposomes with membranes comprising the lipid material.

6. Process for preparing liposomes according to Claim 5 wherein the surface-active agent is a salt of cholic acid.

## Patentansprüche

1. Hämoglobinhaltige Liposome, erhältlich durch Auflösen eines liposommembranbildenden Lipidmaterials und eines Netzmittels in einem organischen Lösungsmittel, Entfernen des Lösungsmittels aus der Lösung und Zugeben einer physiologischen Kochsalzlösung von Hämoglobin, der ein eine Methämoglobinbildung hemmendes Mittel zugesetzt wurde, zum Rest, dadurch gekennzeichnet, daß die derart erhaltene Ausgangslösung zur Entfernung des darin enthaltenen Netzmittels einer Dialyse mit einer physiologischen Kochsalzlösung, die dasselbe eine Methämoglobinbildung hemmende Mittel enthält, unterworfen wird, wobei eine Einkapselung des Hämoglobins und des die Methämoglobinbildung hemmenden Mittels in die Liposomen mit das Lipidmaterial umfassenden Membranen erfolgt.

2. Liposome nach Anspruch 1, dadurch gekennzeichnet, daß es sich bei dem Lipidmaterial um ein Phospholipidmaterial, ausgewählt aus Lecithin, Phosphatidylethanolamin, Phosphatidinsäure, Phosphatidylserin, Phosphatidylinosit, Phosphatidylglycerin, Sphingomyelin, Cardiolipin und deren Hydrierungsprodukten, handelt.

3. Liposome nach Anspruch 1 oder 2, dadurch gekennzeichnet, daß es sich bei dem eine Methämoglobinbildung hemmenden Mittel um ein Salz der Ascorbinsäure oder Glutathion handelt.

4. Liposome nach Ansprüchen 1 bis 3, dadurch gekennzeichnet, daß die Konzentration des in den Liposomen enthaltenen Hämoglobins im Bereich von 5—45% (W/W) beträgt und die Menge an dem verwendeten, die Methämoglobinbildung hemmenden Mittel im Bereich der 2- bis 10fachen molaren Menge des Hämoglobins liegt.

5. Verfahren zur Herstellung hämoglobinhaltiger Liposome durch Auflösen eines liposommembranbildenden Lipidmaterials und eines Netzmittels in einem organischen Lösungsmittel, Entfernen des Lösungsmittels aus der Lösung, Zugabe einer physiologischen Kochsalzlösung von Hämoglobin, der ein eine Methämoglobinbildung hemmendes Mittel zugesetzt wurde, zum Rest und Dialysieren der derart erhaltenen Ausgangslösung mit einer physiologischen Kochsalzlösung, die dasselbe eine Methämoglobinbildung hemmende Mittel enthält, um das Netzmittel aus der Ausgangslösung zu entfernen und dabei das Hämoglobin und das die Methämoglobinbildung hemmende Mittel in den Liposomen mit Membranen, umfassend das Lipidmaterial, einzukapseln.

6. Verfahren zur Herstellung von Liposomen nach Anspruch 5, dadurch gekennzeichnet, daß es sich bei dem Netzmittel um ein Salz der Cholinsäure handelt.

## Revendications

1. Liposomes contenant de l'hémoglobine qui peuvent être obtenus en dissolvant un produit lipidique formateur de membrane des liposomes et un agent tensioactif dans un solvant organique, en retirant le solvant de ladite solution, en ajoutant au résidu une solution physiologique d'hémoglobine à laquelle a été ajouté un agent inhibant la formation de méthémoglobine, caractérisés en ce que la solution de départ ainsi obtenue est soumise à une dialyse avec une solution physiologique contenant le même agent inhibant la formation de méthémoglobine pour retirer l'agent tensioactif de la solution de départ, de sorte que l'hémoglobine et l'agent inhibant la formation de méthémoglobine sont encapsulés dans les liposomes par des membranes comprenant le produit lipidique.

2. Liposomes selon la revendication 1, dans lesquels le produit lipidique est un produit phospholipidique choisi parmi la lécithine, la phosphatidyléthanolamine, l'acide phosphatidique, la phosphatidylsérine, le phosphatidylinositol, le phosphatidylglycérol, la sphingomyéline, la cardiolipine et les produits d'hydrogénation de ceux-ci.

3. Liposomes selon la revendication 1 ou 2, dans lesquels l'agent inhibant la formation de méthémoglobine est un sel de l'acide ascorbique ou le glutathion.

4. Liposomes selon les revendications 1 à 3, dans lesquels la concentration de l'hémoglobine contenue dans les liposomes est comprise dans la gamme de 5 à 45% (poids/poids) et une quantité de l'agent inhibant la formation de méthémoglobine utilisé est comprise dans la gamme de 2 à 10 fois la quantité molaire de l'hémoglobine.

5. Procédé de préparation de liposomes contenant de l'hémoglobine qui comprend la dissolution d'un produit lipidique formateur de membrane de liposomes et d'un agent tensioactif dans un solvant organique, le retrait du solvant de ladite solution, l'addition au résidu d'une solution physiologique d'hémoglobine à laquelle a été ajouté un agent inhibant la formation de méthémoglobine et la soumission de la solution de départ ainsi obtenue à une dialyse avec une solution physiologique contenant le même agent inhibant la formation de méthémoglobine pour retirer lagent tensioactif de la solution de départ, de sorte que l'hémoglobine et l'agent inhibant la formation de méthémoglobine sont encapsulés dans les liposomes par des membranes comprenant le produit lipidique.

6. Procédé de préparation de liposomes selon la revendication 5, dans lequel l'agent tensioactif est un sel de l'acide cholique.